# EUROPEAN PATENT SPECIFICATION

(11) **EP 4 243 770 B1**
(45) Date of publication and mention of the grant of the patent: **30.09.2026**
(21) Application number: 21824259.2
(22) Date of filing: 15.11.2021
(51) Int. Cl.: A61K 9/00, A61K 9/08, A61K 47/10, A61M 5/315

(54) **FORMULATIONS, METHODS, AND PRE-FILLED MULTI-DOSE INJECTION DEVICES WITHOUT CLOUD POINT**
FORMULIERUNGEN, VERFAHREN UND VORGEFÜLLTE MEHRFACHDOSISINJEKTIONSVORRICHTUNGEN OHNE TRÜBUNGSPUNKT
FORMULATIONS, PROCÉDÉS ET DISPOSITIFS D'INJECTION MULTI-DOSE PRÉ-REMPLIS SANS POINT DE TROUBLE

(30) Priority: 16.11.2020 US 202063114187 P
(43) Date of publication of application: 20.09.2023
(73) Proprietor: W. L. Gore & Associates, Inc., Newark, DE 19711 (US)
(72) Inventor: GUNZEL, Edward C., Newark, Delaware 19711 (US); PATAPOFF, Thomas W., Newark, Delaware 19711 (US)
(74) Representative: HGF
(86) International application number: PCT/US2021/072414
(87) International publication number: WO 2022/104389

(56) References cited:
- WO-A1-2017/123840
- WO-A1-2019/173083
- WO-A2-2011/119487
- HADDOU B ET AL: "Cloud point extraction of phenol and benzyl alcohol from aqueous stream", SEPARATION AND PURIFICATION TECHNOLOGY, ELSEVIER SCIENCE, AMSTERDAM, NL, vol. 50, no. 1, 1 June 2006 (2006-06-01), pages 114 - 121, XP028035355, ISSN: 1383-5866, [retrieved on 20060601], DOI: 10.1016/J.SEPPUR.2005.11.014

## Description

### FIELD

The present disclosure relates generally to pre-filled multi-dose injection devices, and in particular, to pre-filled multi-dose injection devices and related therapeutic formulations and methods that lack a cloud point.

### BACKGROUND

Pre-filled injection devices function to both store and deliver therapeutic formulations including drugs and/or biologics. Pre-filled injection devices generally offer cost savings to the pharmaceutical industry and may improve the safety, convenience, and efficacy of drug delivery. Biopharmaceuticals are an important class of pharmaceuticals that may increase the use of pre-filled injection devices, including syringes, and auto injectors. As more pharmaceuticals and particularly biopharmaceuticals are utilized for delivery in pre-filled injection devices, the use of conventional pre-filled technology presents several challenges.

One challenge is the use of silicone (e.g., silicone oil) and/or other liquid lubricants. Conventionally, silicone provides a liquid seal between the stopper and the barrel. While silicone has traditionally been used to ensure that the force required to actuate a pre-filled injection device is minimized, the use of silicone as a lubricant poses a contamination risk. For example, silicone may contaminate the drug or biologic within the injection device. Additionally, the silicone may be injected into a patient along with the drug. Silicone may be of particular concern with biopharmaceuticals because it can cause aggregation of certain proteins, thereby rendering the biopharmaceutical unusable for injection.

Another challenge is the use of polysorbate and/or other surfactants that contain fatty acid esters. Conventionally, surfactants reduce the effect of protein adsorption to the silicone oil and/or reduce interfacial tension. However, such surfactants may interact with preservatives and other excipients and cause the formulation to undergo a phase separation at a cloud point, transitioning from a transparent solution to a turbid solution. In practice, this turbidity may be indistinguishable from bacterial contamination, thus rendering the therapeutic formulation unusable.

Therefore, a need exists for formulations, methods, and pre-filled multi-dose injection devices that lack a cloud point.

### SUMMARY

The present disclosure is directed to pre-filled multi-dose injection devices and related therapeutic formulations and methods that lack a cloud point. The therapeutic formulation may be a transparent solution, and preservatives may remain within the aqueous phase without being phase separated to retain antimicrobial effectiveness.

Features of the present disclosure are set forth in the appended Claims.

The foregoing Examples are just that and should not be read to limit or otherwise narrow the scope of any of the inventive concepts otherwise provided by the instant disclosure. While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The present invention will be better understood in view of the following non-limiting figures, in which:
FIG. 1 is an elevational view of an exemplary pre-filled syringe including a barrel, an actuator, a stopper, a needle, and a therapeutic formulation in accordance with at least one embodiment;
FIG. 2 is a partial cutaway view of the stopper of FIG. 1 having an elastomeric body at least partially covered by a solid lubricant layer in accordance with at least one embodiment;
FIG. 3 is a partial cutaway view of another stopper having an elastomeric body, an intermediate porous layer, and a solid lubricant in accordance with at least one embodiment; and
FIG. 4 is a schematic view of the therapeutic formulation of FIG. 1 in accordance with at least one embodiment.

### DETAILED DESCRIPTION

### Definitions and Terminology

This disclosure is not meant to be read in a restrictive manner. For example, the terminology used in the application should be read broadly in the context of the meaning those in the field would attribute such terminology.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

### Description of Various Embodiments

Persons skilled in the art will readily appreciate that various aspects of the present disclosure can be realized by any number of methods and apparatus configured to perform the intended functions. It should also be noted that the accompanying drawing figures referred to herein are not necessarily drawn to scale and may be exaggerated to illustrate various aspects of the present disclosure, and in that regard, the figures should not be construed as limiting.

### I. Multi-dose Injection Device

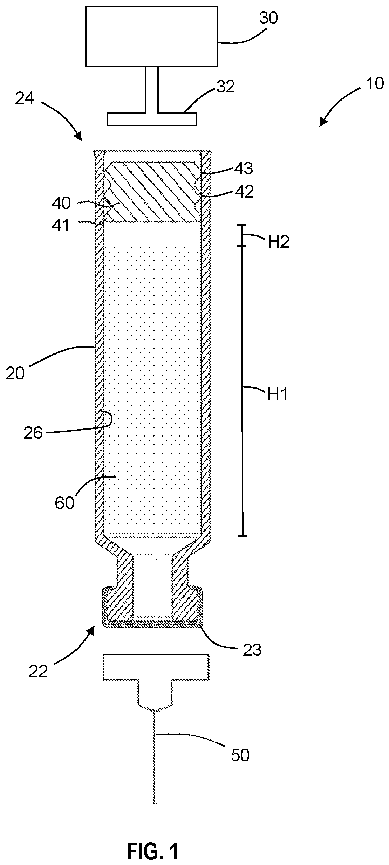

FIG. 1 depicts a drug injection device 10 that may be pre-filled for storing and delivering at least one therapeutic formulation 60 to a patient. The illustrative device 10 includes a housing (not shown), a cartridge or barrel 20, an actuator 30, a stopper 40, and a piercing element (e.g., needle) 50, each of which is described further below. The device 10 may be in the form of an auto-injector or an injectable pen, for example.

The barrel 20 of the device 10 contains the liquid therapeutic formulation 60. The barrel 20 is removably coupled to the housing and includes a distal end 22 that faces toward the patient, a proximal end 24 that faces away from the patient, and an inner surface 26 that faces inward toward the liquid therapeutic formulation 60. In FIG. 1, the distal end 22 of the barrel 20 is covered by a pierceable cap 23. The proximal end 24 of the barrel 20 may also be covered until it is loaded into the housing and/or exposed to the actuator 30. The barrel 20 may be formed of a hard material, such as a glass material (e.g., borosilicate glass), a ceramic material, one or more polymeric materials (e.g., polypropylene, polyethylene, and copolymers thereof), a metallic material, a plastic material (e.g., cyclic olefin polymers and cyclic olefin copolymers), and combinations thereof. In some embodiments, the barrel 20 has already been pre-filled with the therapeutic formulation 60 upon delivery to the user. The barrel 20 may contain about 0.5 mL to about 20 mL of the therapeutic formulation 60, but the device 10 may also be appropriately scaled to smaller doses or larger, multi-doses.

The actuator 30 is removably coupled to the proximal end 24 of the barrel 20. The actuator 30 may include a plunger 32 that is movable within the barrel 20 to discharge the therapeutic formulation 60 by moving the stopper 40. The actuator 30 may include a dose selector (e.g., a dial) (not shown) that controls the distance traveled by the plunger 32 and a trigger (e.g., a button) (not shown) that initiates movement of the plunger 32. The stopper 40 contacts the inner surface 26 of the barrel 20 via one or more sealing ribs 41, 42, 43, although any number of sealing ribs and/or non-sealing ribs may be present on the stopper 40.

As shown in FIG. 1, the stopper 40 may be positioned at a predetermined location in the barrel 20 relative to the therapeutic formulation 60. The therapeutic formulation 60 has a liquid height H1, which depends on the volume of the therapeutic formulation 60 in the barrel 20. The stopper 40 may be located at a predetermined stopper height or "headspace" H2 above the therapeutic formulation 60, which may be measured from the top surface of the therapeutic formulation 60 to the nearest sealing rib 41 of the stopper 40. Although the illustrative stopper 40 of FIG. 1 has a flat bottom surface near sealing rib 41, it is also within the scope of the present disclosure for the bottom surface to have a conical shape that extends downward past the sealing rib 41. The headspace H2 may be selected to control the amount of air in the barrel 20 between the stopper 40 and the therapeutic formulation 60. In some embodiments, the headspace H2 is less than about 25 mm, less than about 23 mm, less than about 21 mm, less than about 19 mm, less than about 17 mm, less than about 15 mm, less than about 13 mm, less than about 10 mm, less than about 8 mm, less than about 5 mm, less than about 3 mm, less than about 2 mm, less than about 1 mm, or less than about 0.5 mm. The headspace volume may be calculated by multiplying the headspace height H2 by the interior cross-sectional area of the barrel 20, less any volume of the stopper 40 that extends past the sealing rib 41 of the stopper 40 toward the therapeutic formulation 60. It may be advantageous to minimize the headspace H2 to reduce or avoid aggregation of the therapeutic formulation 60 in the device 10.

The stopper 40 should have low air and liquid permeability to minimize liquid leakage within the barrel 20 and the introduction of air between the stopper 40 and the inner surface 26 of the barrel 20 when charging or discharging the therapeutic formulation 60. In this way, the stopper 40 may resist bacterial contamination in the barrel 20. The stopper 40 should also possess low-friction slidability relative to the barrel 20 to facilitate the charging and discharging of the therapeutic formulation 60 inside the barrel 20. In some embodiments, the slide force between the stopper 40 and the barrel 20 may be less than 15 N, less than 10 N, or less than 5 N. The stopper 40 is described further in **Section II** below.

The needle 50 of the device 10 is removably coupled to the distal end 22 of the barrel 20, such as using a Luer system. The needle 50 is configured to pierce the patient's skin and inject the therapeutic formulation 60 into the patient when operating the actuator 30. Care should be taken to minimize any bacterial contamination in the barrel 20 when coupling the needle 50 to the barrel 20 and/or when uncoupling the needle 50 from the barrel 20.

The interior of the device 10 (not including the needle 50, as explained below) is free of liquid lubricants (i.e., "lubricant free")or the liquid lubricants are present at a concentration of about 5 µg or less. In particular, the barrel 20 and the stopper 40 of the device 10 are free of silicone (e.g., silicone oil, silicone grease) or the silicone is present at a concentration of about 5 µg or less. As used herein, the phrases "lubricant free" and "free of a liquid lubricant" mean that the barrel 20 and the stopper 40 contain no liquid lubricant of any kind, either intentionally or accidentally (i.e., 0 picograms (pg) of lubricants), or contain only a trace amount of liquid lubricant that is undetectable by any known measuring equipment or method. The phrases "substantially lubricant free" and "substantially free of a liquid lubricant" mean that the barrel 20 and the stopper 40 contain about 5 µg or less of liquid lubricants, optionally, about 4 µg or less, about 3 µg or less, about 2 µg or less, or about 1 µg or less. In certain embodiments, liquid lubricants are present on the barrel 20 and/or the stopper 40 from 0 µg to about 5 µg, from about 1 µg to about 5 µg, from about 2 µg to about 5 µg, from about 3 µg to about 5 µg, or from about 4 µg to about 5 µg. The absence or substantial absence of liquid lubricants can be measured using gas chromatography (GC) mass spectrometry, inductively coupled plasma (ICP) mass spectrometry, and/or by the amount of particles in the barrel 20 that are measured in water for injection (WFI) after the WFI has been exposed to a fully assembled syringe (e.g., a glass barrel 20 and stopper 40 and alternatively at least one therapeutic compound). In some embodiments, the amount of particles in the barrel 20 may be less than about 600 particles/ml for particles greater than 10 pm in size or less than 60 particles/ml for particles greater than 25 pm in size when measured in WFI. The needle 50 of the device 10 may have a lubricant to ease insertion into the patient's skin without impacting the ability for the rest of the device 10 to be free "lubricant free" or "substantially lubricant free", as described above.

### II. Stopper

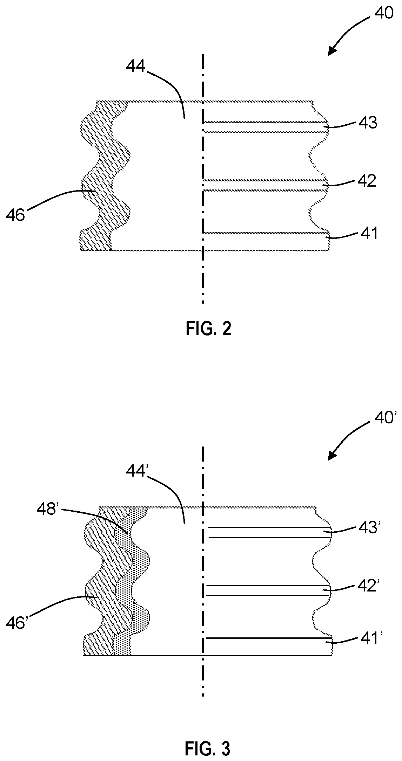

Referring next to FIG. 2, the stopper 40 is shown in more detail and includes an elastomeric body 44 at least partially covered by a solid lubricant layer 46. The solid lubricant layer 46 may be designed to provide a low coefficient of friction with the barrel 20 (FIG. 1), compliance, low extractables and leachables (in particular, low metal-ion extractables and leachables), and/or good barrier properties against any extractables and leachables in the elastomeric body 44.

The elastomeric body 44 of the stopper 40 may comprise any suitable elastomer, such as butyl rubber, bromobutyl rubber, chlorobutyl rubber, silicone, nitrile, styrene butadiene, polychloroprene, ethylene propylene diene, fluoroelastomers and combinations thereof. In other embodiments, the stopper 40 may be constructed of non-elastomeric materials, such as plastics (e.g., polypropylene, polycarbonate, and polyethylene), thermoplastics, and fluoropolymer materials such as ethylene-(perfluoroethylene-propene) copolymer (EFEP), polyvinylidene difluoride (PVDF), and perfluoroalkoxy polymer resin (PFA).

The solid lubricant layer 46 of the stopper 40 may comprise a low coefficient of friction polymer layer, which may have a coefficient of friction of about 0.08 to about 0.8 against the glass of the barrel 20. The solid lubricant layer 46 may be constructed of a fluoropolymer including, but not limited to, polytetrafluoroethylene (PTFE), expanded polytetrafluoroethylene (ePTFE), densified ePTFE, and copolymers and combinations thereof. Other materials for use as the solid lubricant layer 46 include, but are not limited to, fluorinated ethylene propylene (FEP), ethylene tetrafluoroethylene (ETFE), polyvinylfluoride, polyvinylidene fluoride (e.g., poly(vinylidene fluoride-co-tetrafluoroethylene) (VDF-co-TFE), poly(vinylidene fluoride-co-trifluoroethylene) (VDE-co-TrFE)), perfluoropropylvinylether, perfluoroalkoxy polymers, polyethylene (e.g., expanded ultra-high molecular weight polyethylene (eUHMWPE)), polypropylene, poly (*p*-xylylene) (PPX), polylactic acid (PLA), poly(L-lactic acid) (PLLA), poly(D-lactic acid) (PDLA), and copolymers and combinations thereof, which may be expanded if desired.

The stopper 40 of FIG. 2 may be manufactured by thermoforming the solid lubricant layer 46 from a densified ePTFE film and then molding (e.g., injection molding, compression molding) the elastomeric body 44 onto the thermoformed solid lubricant layer 46. In another embodiment, the stopper 40 of FIG. 2 may be manufactured by a direct molding process, in which a sheet of the solid lubricant layer 46 is placed in a heated mold together with the material for elastomeric body 44 to simultaneously vulcanize the elastomeric body 44, if applicable, and form the stopper 40. It is also within the scope of the present disclosure to pre-treat or post-treat the solid lubricant layer 46 with chemical etching, plasma treating, corona treatment, roughening, or the like to improve the bonding of the solid lubricant layer 46 to the elastomeric body 44.

Referring next to FIG. 3, another stopper 40' is shown, with like reference numerals identifying like elements. The stopper 40' includes an elastomeric body 44' and a solid lubricant layer 46' (also referred to as a barrier layer), as well as an intermediate porous layer 48'. The porous layer 48' may comprise or be formed of ePTFE or other porous expanded and advantageously fibrillizing fluoropolymers. The adjacent elastomeric body 44' and/or solid lubricant layer 46' may at least partially penetrate the intermediate porous layer 48', and the degree of penetration may be controlled to achieve desired strength, toughness, compliance and stability for the desired application.

The stopper 40' of FIG. 3 may be manufactured by forming the porous layer 48', coating, laminating, imbibing, or otherwise applying the solid lubricant layer 46' onto and/or into the porous layer 48' to create a multi-layered or composite film, and then molding (e.g., injection molding, compression molding) the elastomeric body 44' onto the film such that the elastomeric body 44' at least partially penetrates the pores of the porous layer 48'. It is also within the scope of the present disclosure to pre-treat or post-treat the solid lubricant layer 46' and/or the porous layer 48' with chemical etching, plasma treating, corona treatment, roughening, or the like to improve the bonding of the solid lubricant layer 46' to the elastomeric body 44'.

### III. Therapeutic Formulation

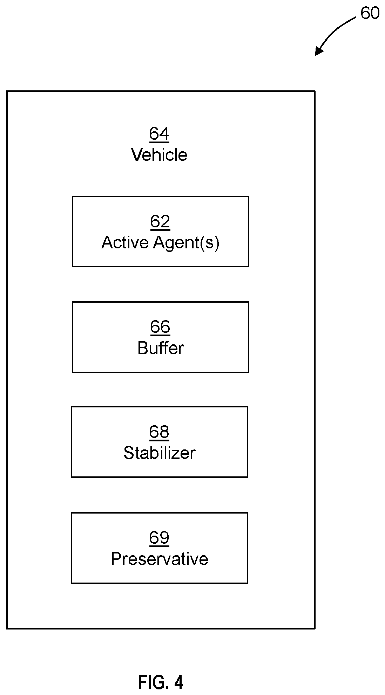

The therapeutic formulation 60 is shown schematically in FIG. 4. The therapeutic formulation 60 may be a multi-dose formulation (which may also be referred to as a multi-use formulation) that is intended to be delivered to the patient in multiple doses over time. As noted above, the multi-dose therapeutic formulation 60 may be present in a larger volume than a comparable single-dose formulation.

The therapeutic formulation 60 includes one or more active pharmacological agents 62, more specifically active biopharmaceuticals agents. The active agents 62 may be used for use in the treatment of inflammatory diseases including, but not limited to, inrheumatoid arthritis (RA), psoriasis, inflammatory bowel disease (IBD), and ocular inflammatory disease. Active agents 62 include, but are not limited to, proteins, antibodies, cytokines, insulin, insulin analogs, growth hormones, growth factors, coagulation factors, proteases, kinases, phosphatases, vaccines, peptides, small interfering RNAs (siRNAs), small interfering DNAs (siDNAs), messenger RNAs (mRNAs), aptamers, and/or any combination thereof. The active agent(s) 62 may be present in the therapeutic formulation 60 at a concentration of at least about 1 mg/ml, such as a concentration from about 1 mg/ml to about 200 mg/ml, from about 10 mg/ml to about 200 mg/ml, from about 20 mg/ml to about 200 mg/ml, from about 40 mg/ml to about 200 mg/ml, from about 60 mg/ml to about 200 mg/ml, from about 80 mg/ml to about 200 mg/ml, from about 100 mg/ml to about 200 mg/ml, from about 120 mg/ml to about 200 mg/ml, and/or from about 150 mg/ml to about 200 mg/ml. Specific active agents 62 are set forth in **Section IV** below.

The therapeutic formulation 60 of FIG. 4 also includes a vehicle 64 (e.g., solvent, diluent) capable of conveying the active agent 62 to the patient during injection. Suitable solvents include, for example, water, acetic acid, propylene glycol, ethylene glycol, polyethylene glycol, benzyl benzoate, and combinations thereof.

The therapeutic formulation 60 may also include one or more excipients. The excipients may be configured to protect, support, or enhance processability, stability, sterility, bioavailability, product identification, effectiveness, delivery, and/or storage integrity.

One excipient is a buffer 66 including, for example, phosphate (e.g., phosphate buffered saline (PBS), acetate, histidine, and tris. The buffer 66 may have a pH from about 4.0 to about 9.5, from about 4.5 to about 9.0, from about 5.0 to about 8.5, from about 5.5 to about 8.0, from about 5.5 to about 7.5, from about 5.5 to about 7.0, and/or from about 5.5 to about 6.5.

Another excipient is a stabilizer 68 including, for example, sugars (e.g., sucrose, trehalose, maltose, and lactose), polyols (e.g., mannitol, sorbitol, and glycerol), and amino acid salts (e.g., histidine, arginine, and glycine). The concentration of sugar in the therapeutic formulation 60 may be from 0 wt.% to about 15 wt.%, from about 0.1 wt.% to about 15 wt.%, from about 1 wt.% to about 15 wt.%, from about 1.5 wt.% to about 10 wt.%, from about 2 wt.% to about 10 wt.%, from about 3 wt.% to about 10 wt.%, and/or from about 5 wt.% to about 10 wt.%. The concentration of polyol in the therapeutic formulation 60 may be from 0 wt.% to about 5 wt.%, from about 0.1 wt.% to about 5 wt.%, from about 1 wt.% to about 5 wt.%, from about 1.5 wt.% to about 5 wt.%, from about 2 wt.% to about 5 wt.%, and/or from about 3 wt.% to about 5 wt.%. The concentration of amino acid salts in the therapeutic formulation 60 may be from 0 wt.% to about 5 wt.%, from about 0.1 wt.% to about 5 wt.%, from about 1 wt.% to about 5 wt.%, from about 1.5 wt.% to about 5 wt.%, from about 2 wt.% to about 5 wt.%, and/or from about 3 wt.% to about 5 wt.%.

Yet another excipient is a preservative 69 having antimicrobial activity. Preservatives include, for example, phenolic preservatives (e.g., phenol, ortho-cresol, meta-cresol, para-cresol, propylparaben, methylparaben), benzyl alcohol preservatives (e.g., methylbenzyl alcohols), and/or any combination thereof. The concentration of preservatives 69 in the therapeutic formulation 60 may be from about 0.1 wt.% to about 5 wt.%, from about 0.1 wt.% to about 3 wt.%, from about 0.1 wt.% to about 1 wt.%, and/or from about 0.2 wt.% to about 0.8 wt.%.

The therapeutic formulation 60 of FIG. 4 is free of polysorbate surfactants (i.e., "polysorbate surfactant free") or the polysorbate surfactant is present at a concentration from 0 wt.% to about 0.1 wt. %(i.e., "substantially polysorbate surfactant free" or "substantially free of a polysorbate surfactant"). In particular, the therapeutic formulation 60 is free of polysorbate surfactant or the polysorbate surfactant is present at a concentration from 0 wt.% to about 0.1 wt. % of polysorbate surfactants, including polysorbate 20, polysorbate 40, polysorbate 60, polysorbate 80, and combinations thereof. Such surfactants are generally used to lower surface tension and/or interfacial tension and prevent therapeutics from adsorbing onto surfaces and/or interfaces. As used herein, the phrase "polysorbate surfactant free" means that the therapeutic formulation 60 contains no polysorbate surfactants , either intentionally or accidentally (i.e., 0 wt.% surfactants), or contains only a trace amount of surfactants that is undetectable by any known measuring equipment or method. The phrase "substantially polysorbate surfactant free" and "substantially free of a polysorbate surfactant" mean that the therapeutic formulation 60 contains an insignificant but measurable amount of polysorbate surfactants at a concentration from about 0.1 wt.% or less; optionally, at a concentration from about 0.075 wt.% or less, about 0.05 wt.% or less, about 0.025 wt.% or less, about 0.01 wt.% or less, about 0.005 wt.% or less, or about 0.001 wt.% or less. In certain embodiments, the concentration of surfactants in the therapeutic formulation 60 may be from 0 wt.% to about 0.1 wt.%, from 0 wt.% to about 0.075 wt.%, from 0 wt.% to about 0.05 wt.%, from 0 wt.% to about 0.025 wt.%, from 0 wt.% to about 0.01 wt.%, from 0 wt.% to about 0.005 wt.%, and/or from 0 wt.% to about 0.001 wt.%.

The therapeutic formulation 60 of FIG. 4 also lacks a cloud point, even when heated to 30 °C, 40 °C, or 50 °C, for example. The therapeutic formulation 60 remains transparent and avoids aggregation, precipitation, or decomposition of the preservative 69, in particular, which would pull the preservative 69 out of the aqueous phase and decrease the antimicrobial activity of the preservative 69. The therapeutic formulation 60 may retain at least about 80% of the preservative 69 in the solution after heating, such as about 80%, about 85%, about 90%, about 95%, about 99%, or about 100%. In terms of turbidity measured with a nephelometer, the therapeutic formulation 60 may exhibit a turbidity increase after heating of 30 nephelometric turbidity units (NTU) or less when heated, such as about 25 NTU, about 20 NTU, about 15 NTU, about 10 NTU, about 5 NTU, about 1 NTU, or about 0 NTU. In terms of apparent absorbance measured at a wavelength of 400nm to 500nm with a spectrometer, the therapeutic formulation 60 may exhibit an absorbance increase after heating of about 0.1 AU or less, such as about 0.1 AU, about 0.08 AU, about 0.06 AU, about 0.04 AU, about 0.02 AU, or about 0 AU.

### IV. Active Agents

As noted above, the therapeutic formulation 60 includes one or more active agents 62, which may include biomolecules such as proteins, antibodies, cytokines, insulin, insulin analogs, growth hormones, growth factors, coagulation factors, proteases, kinases, phosphatases, vaccines, peptides, small interfering RNAs (siRNAs), small interfering DNAs (siDNAs), messenger RNAs (mRNAs), aptamers, and/or any combination thereof.

The invention of this application has been described above both generically and with regard to specific embodiments. It will be apparent to those skilled in the art that various modifications and variations can be made in the embodiments without departing from the scope of the disclosure. Thus, it is intended that the embodiments cover the modifications and variations of this invention provided they come within the scope of the appended claims and their equivalents.

### PROPHETIC EXAMPLES

### Prophetic Example A: Impact of Surfactants on Cloud Point

**Sample Preparation:** Samples containing a buffer (e.g., 20mM histidine chloride buffer, pH 6) will be prepared in 3 cc or larger glass vials. The samples will contain various preservative types (e.g., phenol, methylparaben, meta-cresol) and concentrations (e.g., 0 wt.%, 0.2 wt.%, 0.4 wt.%, 0.6 wt.%, 0.8 wt.%). The samples will also contain various polysorbate surfactants (e.g., polysorbate 20 (PS20) and polysorbate 80 (PS80)) and concentrations (e.g., 0 wt.%, 0.01 wt.%, 0.02 wt.%, 0.05 wt.%, 0.1 wt.%, 0.2 wt.%).

**Heating:** Each sample solution will be placed in a 1cm pathway glass/quartz cuvette with a micro-stir bar and a thermocouple at the top of the solution. Each cuvette will be placed in a spectrophotometer with a temperature-controlled cuvette holder. Each sample will be stirred while equilibrating the solution at 15 °C and then heating the solution 1 °C per minute to 50 °C. Once a cloud point is passed, each sample may be cooled back to 15 °C and the heating repeated.

**Analysis:** One suitable instrument is a spectrophotometer that measures apparent absorption at 400nm - 500nm during the heating process to determine the temperature at which the apparent absorption increases rapidly. Another suitable instrument is a nephelometer that measures turbidity in nephelometric turbidity units (NTU) during the heating process to determine the temperature at which the turbidity increases rapidly.

**Prophetic Results:** For samples without the PS20 or PS80 surfactant, the present inventors believe that the samples will not exhibit a rapid increase in absorption (e.g., 0.1 AU or less) or turbidity (e.g., 30 NTU or less) during heating. For samples with the PS20 or PS80 surfactant, the present inventors believe that the samples will exhibit a rapid increase in absorption (e.g., 0.15 AU or more) or turbidity (e.g., 50 NTU or more), which is indicative of a cloud point.

### Prophetic Example B: Effect of Cloud Point on Antimicrobial Effectiveness

**Sample Preparation:** The same samples from Example A are prepared and placed in 50 cc vials, with each sample having a volume of 30 mL.

**Heating:** Each sample is heated above its cloud point of Example A.

**Centrifugation:** Each clouded sample is centrifuged at that temperature to collect the supernatant and remove the turbid phase.

**Analysis:** Using an extinction coefficient from a standard curve, the supernatant is subjected to spectrophotometry to determine the preservative concentration that remains in the supernatant. Select samples may also be subjected to an Antimicrobial Effectiveness Test (AET) to evaluate the antimicrobial effectiveness of the preservative that is available in the aqueous phase.

**Prophetic Results:** The present inventors believe that the PS20 or PS80 surfactant concentration will have an indirect effect on the preservative concentration in the aqueous phase. For example, the present inventors believe that the samples containing PS20 or PS80 concentrations of 0.01 wt.% or more may lose more than about 20%, about 25%, or about 30%, of the preservative in the aqueous phase. The samples without PS20 or PS80 are expected to avoid phase separation altogether and thus retain all of the of the preservative in the aqueous phase.

## Claims

1. A pre-filled multi-dose injectable device comprising:
a stopper;
a barrel;
a solid lubricant on at least one of the stopper and the barrel, the pre-filled multi-dose injectable device being free of a liquid lubricant or the liquid lubricant is present in an amount of about 5 µg or less (+/- 10% of stated value); and
at least one formulated therapeutic comprising:
an active pharmacological agent; and
at least one phenolic or benzyl alcohol preservative;
wherein the at least one formulated therapeutic is free of polysorbate surfactant or the polysorbate surfactant is present at a concentration from 0 wt.% to about 0.1 wt.% (+/- 10% of stated value), wherein the polysorbate surfactant is selected from at least one of polysorbate 20, polysorbate 40, polysorbate 60, polysorbate 80, or a combination thereof and wherein the at least one formulated therapeutic exhibits a turbidity increase of less than 30 nephelometric turbidity units (NTU) upon warming from 5 °C to 30 °C.

2. The pre-filled multi-dose injectable device of claim 1, wherein the active pharmacological agent comprises at least one of proteins, antibodies, cytokines, insulin, insulin analogs, growth hormones, growth factors, coagulation factors, proteases, kinases, phosphatases, vaccines, peptides, small interfering RNAs (siRNAs), small interfering DNAs (siDNAs), messenger RNAs (mRNAs), aptamers, and/or a combination thereof.

3. The pre-filled multi-dose injectable device of any one of claims 1-2, wherein the at least one formulated therapeutic includes the active pharmacological agent at a concentration from about 1 mg/ml to about 200 mg/ml, from about 10 mg/ml to about 200 mg/ml, from about 20 mg/ml to about 200 mg/ml, from about 50 mg/ml to about 200 mg/ml, from about 80 mg/ml to about 200 mg/ml, from about 100 mg/ml to about 200 mg/ml, from about 120 mg/ml to about 200 mg/ml, and/or from about 150 mg/ml to about 200 mg/ml.

4. The pre-filled multi-dose injectable device of any one of claims 1-3, wherein the at least one formulated therapeutic includes the polysorbate surfactant at a concentration from from 0 wt.% to about 0.075 wt.%, from 0 wt.% to about 0.05 wt.%, from 0 wt.% to about 0.025 wt.%, from 0 wt.% to about 0.01 wt.%, from 0 wt.% to about 0.005 wt.%, and/or from 0 wt.% to about 0.001 wt.%.

5. The pre-filled multi-dose injectable device of any one of claims 1-4, the at least one formulated therapeutic comprises a buffer having a pH from about 4.0 to about 9.5, from about 4.5 to about 9.0, from about 5.0 to about 8.5, from about 5.5 to about 8.0, from about 5.5 to about 7.5, from about 5.5 to about 7.0, and/or from about 5.5 to about 6.5.

6. The pre-filled multi-dose injectable device of any one of claims 1-5, wherein the stopper and the barrel are free of the liquid lubricant silicone.

7. The pre-filled multi-dose injectable device of any one of claims 1-6, wherein the barrel is made of at least one of a glass material, a plastic material, a ceramic material, a metallic material and/or a combination thereof.

8. The pre-filled multi-dose injectable device of any one of claims 1-7, wherein the turbidity increase is from 0 NTU to about 25 NTU, from 0 NTU to about 20 NTU, from 0 NTU to about 15 NTU, from 0 NTU to about 10 NTU, from 0 NTU to about 5 NTU, or from 0 NTU to about 1 NTU.

9. The pre-filled multi-dose injectable device of any one of claims 1-8, wherein the turbidity increase is no more than about 20 NTU, no more than about 10 NTU, no more than about 5 NTU, and/or no more than about 1 NTU.

10. The pre-filled multi-dose injectable device of any one of claims 1-9, wherein the solid lubricant is a low coefficient of friction layer and the stopper comprises an elastomeric body and the low coefficient of friction layer positioned on the elastomeric body.

11. The pre-filled multi-dose injectable device of claim 10, wherein the low coefficient of friction layer comprises a fluoropolymer.

12. The pre-filled multi-dose injectable device of claim 11, wherein the fluoropolymer of the low coefficient of friction layer is an expanded fluoropolymer.

13. The pre-filled multi-dose injectable device of claim 12, wherein the elastomeric body is at least partially imbibed into the expanded fluoropolymer of the low coefficient of friction layer.

14. The pre-filled multi-dose injectable device of any one of claims 12-13, wherein the expanded fluoropolymer of the low coefficient of friction layer is pre-treated with at least one treatment of chemical etching, plasma treating, corona, and physical modification.

15. The pre-filled multi-dose injectable device of any one of claims 11-14, wherein the fluoropolymer of the low coefficient of friction layer is an expanded polytetrafluoroethylene (ePTFE).

## Patentansprüche

1. Vorgefüllte Mehrfachdosisinjektionslösungsvorrichtung, umfassend:
einen Stopper;
einen Zylinder;
ein festes Schmiermittel auf zumindest einem von dem Stopper und dem Zylinder, wobei die vorgefüllte Mehrfachdosisinjektionslösungsvorrichtung frei von einem flüssigen Schmiermittel ist oder das flüssige Schmiermittel in einer Menge von etwa 5 µg oder weniger (+/-10 % des angegebenen Wertes) vorhanden ist; und
zumindest ein formuliertes Therapeutikum, umfassend:
ein aktives pharmakologisches Mittel; und
zumindest ein Phenol- oder Benzylalkohol-Konservierungsmittel;
wobei das zumindest eine formulierte Therapeutikum frei von Polysorbat-Tensid ist oder das Polysorbat-Tensid in einer Konzentration von 0 Gew.-% bis etwa 0,1 Gew.-% (+/-10 % des angegebenen Wertes) vorhanden ist,
wobei das Polysorbat-Tensid ausgewählt ist aus zumindest einem von Polysorbat 20, Polysorbat 40, Polysorbat 60, Polysorbat 80 oder einer Kombination davon und wobei das zumindest eine formulierte Therapeutikum eine Trübheitserhöhung von weniger als 30 nephelometrischen Trübheitseinheiten (NTU) bei Erwärmung von 5 °C auf 30 °C aufweist.

2. Vorgefüllte Mehrfachdosisinjektionslösungsvorrichtung nach Anspruch 1, wobei das aktive pharmakologische Mittel zumindest eines von Proteinen, Antikörpern, Zytokinen, Insulin, Insulinanaloga, Wachstumshormonen, Wachstumsfaktoren, Gerinnungsfaktoren, Proteasen, Kinasen, Phosphatasen, Impfstoffen, Peptiden, kleinen interferierenden RNAs (siRNAs), kleinen interferierenden DNAs (siDNAs), Boten-RNAs (mRNAs), Aptameren und/oder einer Kombination davon umfasst.

3. Vorgefüllte Mehrfachdosisinjektionslösungsvorrichtung nach einem der Ansprüche 1-2, wobei das zumindest eine formulierte Therapeutikum das aktive pharmakologische Mittel in einer Konzentration von etwa 1 mg/ml bis etwa 200 mg/ml, von etwa 10 mg/ml bis etwa 200 mg/ml, von etwa 20 mg/ml bis etwa 200 mg/ml, von etwa 50 mg/ml bis etwa 200 mg/ml, von etwa 80 mg/ml bis etwa 200 mg/ml, von etwa 100 mg/ml bis etwa 200 mg/ml, von etwa 120 mg/ml bis etwa 200 mg/ml und/oder von etwa 150 mg/ml bis etwa 200 mg/ml beinhaltet.

4. Vorgefüllte Mehrfachdosisinjektionslösungsvorrichtung nach einem der Ansprüche 1-3, wobei das zumindest eine formulierte Therapeutikum das Polysorbat-Tensid in einer Konzentration von 0 Gew.-% bis etwa 0,075 Gew.-%, von 0 Gew.-% bis etwa 0,05 Gew.-%, von 0 Gew.-% bis etwa 0,025 Gew.-%, von 0 Gew.-% bis etwa 0,01 Gew.-%, von 0 Gew.-% bis etwa 0,005 Gew.-% und/oder von 0 Gew.-% bis etwa 0,001 Gew.-% beinhaltet.

5. Vorgefüllte Mehrfachdosisinjektionslösungsvorrichtung nach einem der Ansprüche 1-4, das zumindest eine formulierte Therapeutikum einen Puffer mit einem pH von etwa 4,0 bis etwa 9,5, von etwa 4,5 bis etwa 9,0, von etwa 5,0 bis etwa 8,5, von etwa 5,5 bis etwa 8,0, von etwa 5,5 bis etwa 7,5, von etwa 5,5 bis etwa 7,0 und/oder von etwa 5,5 bis etwa 6,5 umfasst.

6. Vorgefüllte Mehrfachdosisinjektionslösungsvorrichtung nach einem der Ansprüche 1-5, wobei der Stopper und der Zylinder frei von dem flüssigen Schmiermittel Silikon sind.

7. Vorgefüllte Mehrfachdosisinjektionslösungsvorrichtung nach einem der Ansprüche 1-6, wobei der Zylinder aus zumindest einem von einem Glasmaterial, einem Kunststoffmaterial, einem Keramikmaterial, einem metallischen Material und/oder einer Kombination davon hergestellt ist.

8. Vorgefüllte Mehrfachdosisinjektionslösungsvorrichtung nach einem der Ansprüche 1-7, wobei die Trübheitserhöhung von 0 NTU bis etwa 25 NTU, von 0 NTU bis etwa 20 NTU, von 0 NTU bis etwa 15 NTU, von 0 NTU bis etwa 10 NTU, von 0 NTU bis etwa 5 NTU oder von 0 NTU bis etwa 1 NTU ist.

9. Vorgefüllte Mehrfachdosisinjektionslösungsvorrichtung nach einem der Ansprüche 1-8, wobei die Trübheitserhöhung nicht mehr als etwa 20 NTU, nicht mehr als etwa 10 NTU, nicht mehr als etwa 5 NTU und/oder nicht mehr als etwa 1 NTU ist.

10. Vorgefüllte Mehrfachdosisinjektionslösungsvorrichtung nach einem der Ansprüche 1-9, wobei das feste Schmiermittel eine Schicht mit niedrigem Reibungskoeffizienten ist und der Stopper einen Elastomerkörper umfasst und die Schicht mit niedrigem Reibungskoeffizienten auf dem Elastomerkörper positioniert ist.

11. Vorgefüllte Mehrfachdosisinjektionslösungsvorrichtung nach Anspruch 10, wobei die Schicht mit niedrigem Reibungskoeffizienten ein Fluorpolymer umfasst.

12. Vorgefüllte Mehrfachdosisinjektionslösungsvorrichtung nach Anspruch 11, wobei das Fluorpolymer der Schicht mit niedrigem Reibungskoeffizienten ein expandiertes Fluorpolymer ist.

13. Vorgefüllte Mehrfachdosisinjektionslösungsvorrichtung nach Anspruch 12, wobei der Elastomerkörper zumindest teilweise in das expandierte Fluorpolymer der Schicht mit niedrigem Reibungskoeffizienten aufgenommen ist.

14. Vorgefüllte Mehrfachdosisinjektionslösungsvorrichtung nach einem der Ansprüche 12-13, wobei das expandierte Fluorpolymer der Schicht mit niedrigem Reibungskoeffizienten mit zumindest einer Behandlung aus chemischem Ätzen, Plasmabehandlung, Korona und physikalischer Modifikation vorbehandelt ist.

15. Vorgefüllte Mehrfachdosisinjektionslösungsvorrichtung nach einem der Ansprüche 11-14, wobei das Fluorpolymer der Schicht mit niedrigem Reibungskoeffizienten ein expandiertes Polytetrafluorethylen (ePTFE) ist.

## Revendications

1. Dispositif injectable multi-dose pré-rempli comprenant :
un bouchon ;
un cylindre ;
un lubrifiant solide sur au moins un parmi le bouchon et le cylindre, le dispositif injectable multi-dose pré-rempli étant exempt de lubrifiant liquide ou le lubrifiant liquide est présent en une quantité d'environ 5 µg ou moins (+/- 10 % de la valeur indiquée) ; et
au moins un produit thérapeutique formulé comprenant :
un agent pharmacologique actif ; et
au moins un conservateur d'alcool phénolique ou benzylique ;
ledit au moins un agent thérapeutique formulé étant exempt de tensioactif polysorbate ou ledit tensioactif polysorbate étant présent à une concentration allant de 0 % en poids à environ 0,1 % en poids (+/- 10 % de la valeur indiquée),
ledit tensioactif polysorbate étant choisi parmi le polysorbate 20, le polysorbate 40, le polysorbate 60, le polysorbate 80 ou une combinaison de ceux-ci et ledit au moins un agent thérapeutique formulé présentant une augmentation de la turbidité inférieure à 30 unités de turbidité néphélométrique (NTU) lors du réchauffement de 5°C à 30°C.

2. Dispositif injectable multi-dose pré-rempli selon la revendication 1, ledit agent pharmacologique actif comprenant au moins un parmi les protéines, les anticorps, les cytokines, l'insuline, les analogues de l'insuline, les hormones de croissance, les facteurs de croissance, les facteurs de coagulation, les protéases, les kinases, les phosphatases, les vaccins, les peptides, les petits ARN interférents (ARNsi), les petits ADN interférents (ADNsi), les ARN messagers (ARNm), les aptamères et/ou une combinaison de ceux-ci.

3. Dispositif injectable multi-dose pré-rempli selon l'une quelconque des revendications 1 à 2, ledit au moins un agent thérapeutique formulé comprenant l'agent pharmacologique actif à une concentration d'environ 1 mg/ml à environ 200 mg/ml, d'environ 10 mg/ml à environ 200 mg/ml, d'environ 20 mg/ml à environ 200 mg/ml, d'environ 50 mg/ml à environ 200 mg/ml, d'environ 80 mg/ml à environ 200 mg/ml, d'environ 100 mg/ml à environ 200 mg/ml, d'environ 120 mg/ml à environ 200 mg/ml, et/ou d'environ 150 mg/ml à environ 200 mg/ml.

4. Dispositif injectable multi-dose pré-rempli selon l'une quelconque des revendications 1 à 3, ledit au moins un agent thérapeutique formulé comprenant le tensioactif polysorbate à une concentration allant de 0 % en poids à environ 0,075 % en poids, de 0 % en poids à environ 0,05 % en poids, de 0 % en poids à environ 0,025 % en poids, de 0 % en poids à environ 0,01 % en poids, de 0 % en poids à environ 0,005 % en poids, et/ou de 0 % en poids à environ 0,001 % en poids.

5. Dispositif injectable multi-dose pré-rempli selon l'une quelconque des revendications 1 à 4, ledit au moins un agent thérapeutique formulé comprenant un tampon présentant un pH d'environ 4,0 à environ 9,5, d'environ 4,5 à environ 9,0, d'environ 5,0 à environ 8,5, d'environ 5,5 à environ 8,0, d'environ 5,5 à environ 7,5, d'environ 5,5 à environ 7,0, et/ou d'environ 5,5 à environ 6,5.

6. Dispositif injectable multi-dose pré-rempli selon l'une quelconque des revendications 1 à 5, ledit bouchon et ledit cylindre étant exempts de silicone lubrifiant liquide.

7. Dispositif injectable multi-dose pré-rempli selon l'une quelconque des revendications 1 à 6, ledit cylindre étant constitué d'au moins un parmi un matériau de verre, un matériau plastique, un matériau céramique, un matériau métallique et/ou une combinaison de ceux-ci.

8. Dispositif injectable multi-dose pré-rempli selon l'une quelconque des revendications 1 à 7, ladite augmentation de la turbidité allant de 0 NTU à environ 25 NTU, de 0 NTU à environ 20 NTU, de 0 NTU à environ 15 NTU, de 0 NTU à environ 10 NTU, de 0 NTU à environ 5 NTU, ou de 0 NTU à environ 1 NTU.

9. Dispositif injectable multi-dose pré-rempli selon l'une quelconque des revendications 1 à 8, ladite augmentation de la turbidité n'étant pas supérieure à environ 20 NTU, pas supérieure à environ 10 NTU, pas supérieure à environ 5 NTU et/ou pas supérieure à environ 1 NTU.

10. Dispositif injectable multi-dose pré-rempli selon l'une quelconque des revendications 1 à 9, ledit lubrifiant solide étant une couche à faible coefficient de frottement et ledit bouchon comprenant un corps élastomère et ladite couche à faible coefficient de frottement étant positionnée sur le corps élastomère.

11. Dispositif injectable multi-dose pré-rempli selon la revendication 10, ladite couche à faible coefficient de frottement comprenant un polymère fluoré.

12. Dispositif injectable multi-dose pré-rempli selon la revendication 11, ledit polymère fluoré de la couche à faible coefficient de frottement étant un polymère fluoré expansé.

13. Dispositif injectable multi-dose pré-rempli selon la revendication 12, ledit corps élastomère étant au moins partiellement imbibé dans le polymère fluoré expansé de la couche à faible coefficient de frottement.

14. Dispositif injectable multi-dose pré-rempli selon l'une quelconque des revendications 12 à 13, ledit polymère fluoré expansé de la couche à faible coefficient de frottement étant pré-traité avec au moins un traitement par gravure chimique, par traitement au plasma, par effet corona et par modification physique.

15. Dispositif injectable multi-dose pré-rempli selon l'une quelconque des revendications 11 à 14, ledit polymère fluoré de la couche à faible coefficient de frottement étant un polytétrafluoroéthylène expansé (PTFEe).
